(19) Europäisches Patentamt

European Patent Office

Office européen des brevets

(11) **EP 1 087 804 B1**

(12) **FASCICULE DE BREVET EUROPEEN**

(45) Date de publication et mention
de la délivrance du brevet:
**02.11.2005 Bulletin 2005/44**

(51) Int Cl.⁷: **A61M 1/16**

(21) Numéro de dépôt: **99925217.4**

(22) Date de dépôt: **17.06.1999**

(86) Numéro de dépôt international:
**PCT/IB1999/001138**

(87) Numéro de publication internationale:
**WO 1999/065543 (23.12.1999 Gazette 1999/51)**

(54) **MACHINE DE DIALYSE, EN PARTICULIER POUR UNE UTILISATION A DOMICILE**

DIALYSEMASCHINE, INSBESONDERE FÜR DEN HAUSGEBRAUCH

DIALYSIS MACHINE, IN PARTICULAR FOR HOME USE

(84) Etats contractants désignés:
**AT BE CH DE DK ES FI FR GB GR IE IT LI LU NL
PT SE**

(30) Priorité: **17.06.1998 FR 9807871**

(43) Date de publication de la demande:
**04.04.2001 Bulletin 2001/14**

(73) Titulaire: **Valemont Participation Corp.
Road Town, Tortola (VG)**

(72) Inventeur: **TRAEGER, Jules
F-69006 Lyon (FR)**

(74) Mandataire: **Nithardt, Roland
Cabinet Roland Nithardt,
Conseils en Propriété Industrielle S.A.,
Y-Parc,
Rue Galilée 9
1400 Yverdon-les-Bains (CH)**

(56) Documents cités:
WO-A-95/20985          US-A- 4 132 644
US-A- 4 386 634        US-A- 4 950 395

## Description

La présente invention concerne une machine de dialyse comportant un dialyseur ayant une première entrée et une première sortie du circuit sanguin, des moyens d'approvisionnement en dialysat, une ligne artérielle connectée entre une fistule artérioveineuse du patient et ladite première entrée du dialyseur, une ligne veineuse connectée entre ladite fistule artérioveineuse et ladite première sortie du dialyseur, une pompe couplée à ladite ligne artérielle, une ligne d'amenée du dialysat, connectée entre lesdits moyens d'approvisionnement en dialysat et une seconde entrée du dialyseur, et une ligne d'évacuation du dialysat, connectée entre un récipient de récupération du dialysat et une seconde sortie du dialyseur.

## Technique antérieure

La dialyse chronique requiert traditionnellement des quantités importantes de dialysat. Des volumes de l'ordre de 120 litres de dialysat sont nécessaires pour permettre des échanges suffisants par diffusion au niveau de la membrane du dialyseur et pour ainsi épurer le sang du patient malade.

Actuellement, la dialyse s'effectue trois fois par semaine à l'hôpital pendant environ quatre heures et nécessite des machines complexes qui comprennent notamment des équipements de traitement de l'eau pour délivrer une eau d'une qualité suffisante sur le plan minéral et sur le plan bactériologique. Cette eau purifiée est additionnée à un concentré pour fabriquer le dialysat requis. Malgré cela et malgré la désinfection journalière des machines de dialyse, les risques de contamination restent importants.

Le fait de traiter le patient à intervalles relativement longs constitue un inconvénient important. Il devient hypercatabolique entre les séances et son traitement nécessite une intervention longue et fatigante pour lui et un appareillage complexe en raison notamment des grandes quantités de dialysat à préparer. Les machines permettant d'effectuer une hémodialyse en milieu hospitalier sont très encombrantes et impossibles à mettre en service par un personnel non qualifié. Il est de ce fait très difficile de laisser un patient se connecter tout seul sans assistance et sans surveillance médicale. La complexité des machines actuelles impose la proximité de techniciens capables d'assurer leur maintenance.

On connaît déjà des machines de dialyse du type tel que décrit en préambule et prévues en particulier pour une utilisation à domicile, notamment par le brevet américain US-A-4 950 395. Ce document décrit un appareil de dialyse très élémentaire qui fonctionne par gravité et la pression sanguine, et par une pompe montée en série sur la ligne d'amenée du dialysat. L'appareil comporte des pinces ou "clamps" montées sur les différentes lignes pour régler à l'aide d'un fluxmètre le débit de dialysat au cours de la séance de dialyse. On constate une importante variation du débit du dialysat entre le début et la fin de la séance qui affecte beaucoup la performance de la dialyse. De plus, le réglage du débit est relativement difficile et nécessite l'assistance continue d'une tierce personne.

La demande internationale de brevet publiée sous le numéro WO-95 20 985 concerne un appareil de dialyse qui comprend principalement une enceinte, une pompe réversible pour pressuriser ou dépressuriser l'enceinte, et une poche chauffante de dialysat stérile et une poche d'évacuation de dialysat utilisé placées dans l'enceinte et connectées par un raccord en T à un cathéter relié au péritoine d'un patient. Un dispositif à clapets permet d'établir sélectivement une communication de liquide entre le cathéter et l'une des poches. Lorsque l'enceinte est dépressurisée, du dialysat stérile s'écoule dans la poche chauffante depuis des poches d'approvisionnement, et du dialysat utilisé est évacué du patient dans la poche d'évacuation. Lorsque l'enceinte est pressurisée, du dialysat stérile et chauffé s'écoule vers le patient depuis la poche chauffante et le dialysat utilisé s'écoule vers un récipient de récupération. Cependant, cet appareil de dialyse est conçu uniquement pour effectuer une dialyse péritonéale.

Le brevet américain US-A-4 132 644 a pour objet un appareil de dialyse à recyclage du dialysat utilisé dont l'écoulement du dialysat est généré par une pompe péristaltique, ce qui oblige à régler le débit du dialysat au cours de la séance. La poche d'alimentation en dialysat est logée dans une enceinte étanche à pression variable pour modifier la pression dans la poche et dans le circuit de manière à réguler le débit du dialysat. Cet appareil est difficile à utiliser correctement et nécessite l'assistance d'une personne qualifiée.

Le brevet américain US-A-4 386 634 a pour objet un appareil et une technique de préparation de dialysat.

## Exposé de l'invention

La présente invention se propose de pallier les divers inconvénients mentionnés précédemment pour une dialyse chronique en permettant de supprimer les séances longues de traitement en milieu hospitalier pour leur substituer des séances de traitement quotidiennes beaucoup plus courtes, ne nécessitant plus que des volumes de dialysat réduits, par exemple conditionnés en sacs stériles, et supprimant le risque pour le patient de devenir hypercatabolique entre deux séances de dialyse, ces séances pouvant être faites à domicile et ne nécessitant aucune assistance ou surveillance médicale et aucune manipulation complexe pour faire fonctionner la machine de dialyse. Un autre but de l'invention est de fournir une machine de dialyse simple et fiable qui peut être utilisée notamment pour une dialyse aiguë, où le traitement s'effectue en continu, et pour pratiquer de l'hémodiafiltration lors de l'utilisation de la machine soit pour une dialyse chronique soit pour une dialyse aiguë.

Ces buts sont atteints par une machine de dialyse telle que décrite en préambule et caractérisée en ce que lesdits moyens d'approvisionnement en dialysat comportent au moins une enceinte dans laquelle est disposée au moins une poche contenant du dialysat stérile, cette poche étant connectée à ladite ligne d'amenée du dialysat vers le dialyseur, et des moyens de mise sous pression de ladite poche pour provoquer l'écoulement du dialysat dans cette ligne d'amenée, en ce que ladite ligne d'amenée de dialysat comporte une restriction de débit calibrée agencée pour générer une perte de charge déterminée de telle façon que, pour une pression déterminée dans l'enceinte, le débit et la pression du dialysat à ladite seconde entrée du dialyseur ont une valeur donnée constante, et en ce que la machine comporte un dispositif volumétrique connecté sur la ligne d'évacuation à la seconde sortie du dialyseur, ce dispositif volumétrique étant agencé pour générer, en combinaison avec lesdits moyens de mises sous pression de l'enceinte, une pression transmembranaire TMP déterminée dans te dialyseur.

Selon une forme de réalisation préférée de la machine de dialyse pour une dialyse aiguë, lesdits moyens d'approvisionnement comportent au moins deux enceintes contenant chacune au moins une poche de dialysat stérile, les deux poches pouvant être mises sous pression séparément par lesdits moyens de mise sous pression, et au moins deux poches de réserve de dialysat stérile disposées à l'extérieur desdites enceintes et connectées chacune à l'une desdites poches respectivement par un conduit. Ladite machine de dialyse comporte avantageusement un système de pinçage alternatif des conduits.

Selon une forme de réalisation particulière de la machine de dialyse pour une utilisation destinée à pratiquer de l'hémodiafiltration, lesdits moyens d'approvisionnement comprennent au moins une poche de dialysat stérile logée dans une enceinte et connectée par l'intermédiaire d'une ligne d'amenée à ladite ligne veineuse.

Lesdits moyens de mise sous pression comportent de préférence au moins un réservoir de gaz raccordé à ladite enceinte. Ils peuvent comporter au moins un compresseur d'air.

De préférence, la ligne d'amenée du dialysat au dialyseur est calibrée pour que, pour une pression déterminée dans l'enceinte, le débit de dialysat ait une valeur sensiblement comprise entre 100 et 500 ml/minute et de préférence comprise entre 100 et 250 ml/minute.

La ligne d'amenée du dialysat peut être constituée au moins en partie d'un tube armé calibré de section constante sur une longueur prédéterminée.

Selon la forme de réalisation particulière de la machine de dialyse pour une utilisation destinée à pratiquer de l'hémodiafiltration, lesdits moyens de mise sous pression maintiennent avantageusement une pression intérieure de l'enceinte suffisante pour provoquer l'écoulement du dialysat dans les lignes d'amenée respectivement à un piège à bulles et au dialyseur. Les lignes d'amenée sont de préférence calibrées pour que, pour une pression déterminée dans l'enceinte, les débits aient une valeur donnée constante.

Ledit dispositif volumétrique peut être une pompe de dialysat dont le débit peut être ajusté.

Selon un mode de construction préféré, la machine de dialyse comporte un bâti portant un logement contenant ledit dialyseur, au moins une enceinte contenant au moins une poche de dialysat stérile, ladite ligne artérielle agencée pour être connectée entre une artère du patient et ladite première entrée du dialyseur, ladite ligne veineuse agencée pour être connectée entre une veine du patient et ladite première sortie du dialyseur, ladite pompe couplée à ladite ligne artérielle, ladite ligne d'amenée du dialysat stérile, connectée entre ladite poche de dialysat stérile et la seconde entrée du dialyseur, ledit récipient de récupération du dialysat, ladite ligne d'évacuation du dialysat, connectée entre ladite seconde sortie du dialyseur et ledit récipient de récupération, et lesdits moyens de mise sous pression de ladite poche de dialysat stérile logée dans ladite enceinte, et un couvercle rabattable attaché à ce bâti.

Selon une forme de construction particulière de la machine de dialyse destinée à pratiquer une dialyse aiguë, ledit logement renferme les deux enceintes contenant chacune au moins une poche de dialysat stérile, et ladite machine de dialyse comporte un support solidaire du bâti et qui porte les poches de réserve de dialysat stérile. Ledit support des poches de réserve peut être constitué par ledit couvercle.

## Description sommaire des dessins

La présente invention sera mieux comprise en référence à la description ci-dessous de différentes formes de réalisation préférées d'une machine de dialyse selon l'invention et en référence aux dessins annexés, donnés à titre d'exemples non limitatifs, dans lesquels:

- la figure 1 représente schématiquement une machine de dialyse selon l'invention, en application sur un patient nécessitant une dialyse chronique,

- les figures 2A et 2B représentent des vues schématiques en perspective

- illustrant les composants essentiels de la machine de dialyse selon l'invention, sous deux formes de réalisation destinées respectivement à pratiquer une dialyse chronique et une dialyse aiguë,

- les figures 3A et 3B représentent des vues éclatées des différents composants de la machine de dialyse correspondant aux deux formes de réalisation illustrées dans les figures 2A et 2B respectivement, et

- les figures 4 et 5 représentent respectivement des vues en élévation frontale et latérale de la machine selon l'invention, illustrant de façon schématique la disposition de ses composants essentiels.

## Meilleure manière de réaliser l'invention

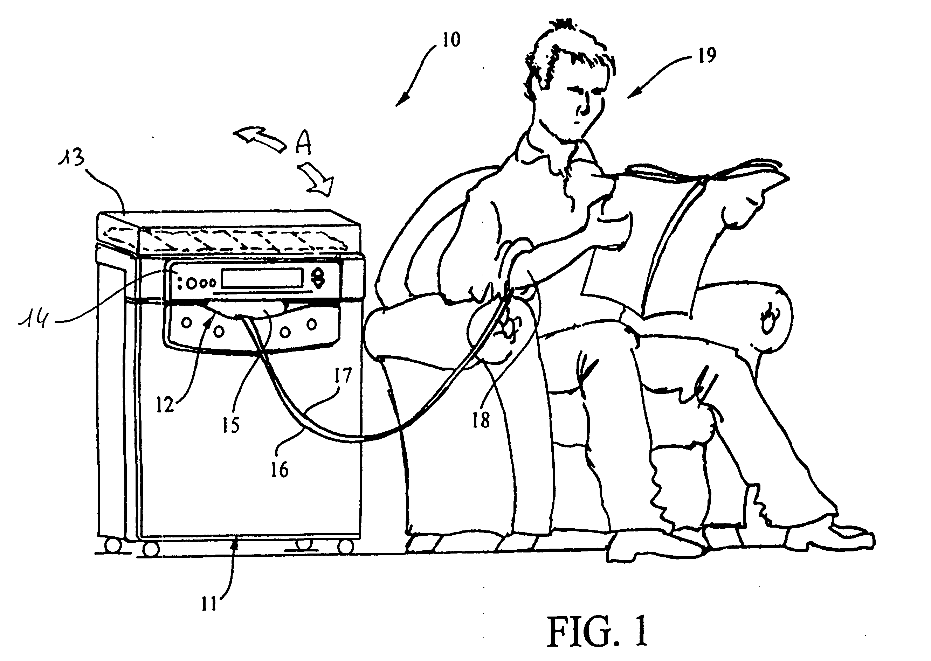

La machine de dialyse 10, telle que représentée schématiquement par la figure 1, comporte principalement un bâti 11 définissant un logement 12 et auquel est attaché un couvercle 13 rabattable, comme le montrent les flèches A. La façade frontale de la machine présente un tableau de commande 14 comportant les boutons de commande requis pour permettre une utilisation simple de la machine par le patient lui-même ou par un assistant qui n'est pas nécessairement très qualifié. Une ouverture 15 ménagée dans le tableau de commande 14 permet le passage de conduits appelés ligne artérielle 16 et ligne veineuse 17 qui se raccordent à une fistule artérioveineuse (non représentée en détail) implantée dans l'avant bras 18 d'un patient 19. Dans le mode d'utilisation représenté sur la figure 1, il s'agit d'une dialyse chronique où le patient doit être dialysé de façon régulière, de préférence quotidiennement. La machine selon l'invention, par sa simplicité de maniement et de commande, permet au patient lui-même d'effectuer l'intervention. Les boutons de commande du tableau peuvent être complétés ou remplacés par une télécommande à rayonnement infrarouge ou similaire.

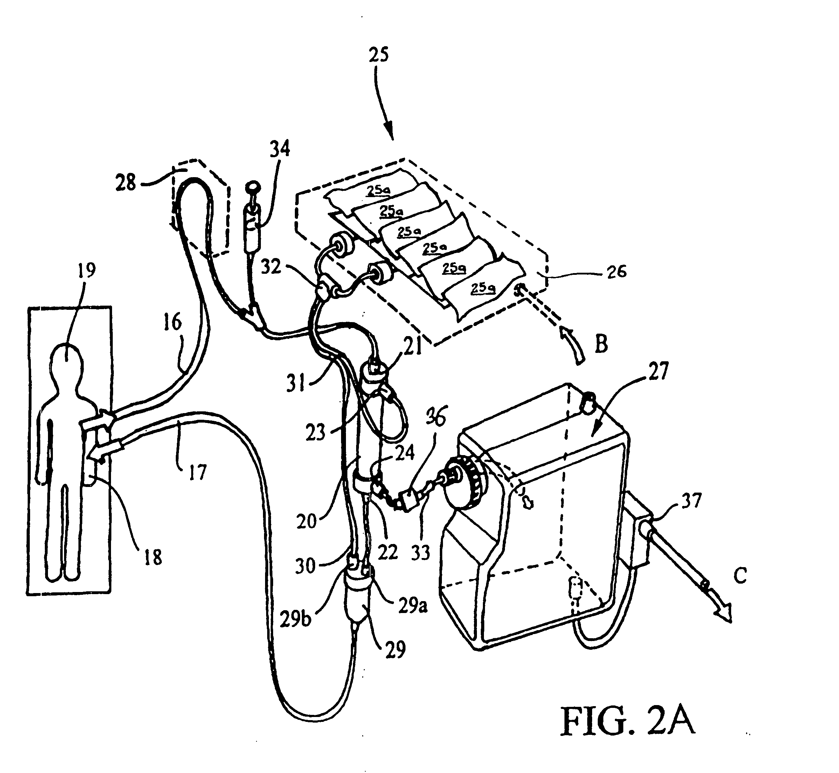

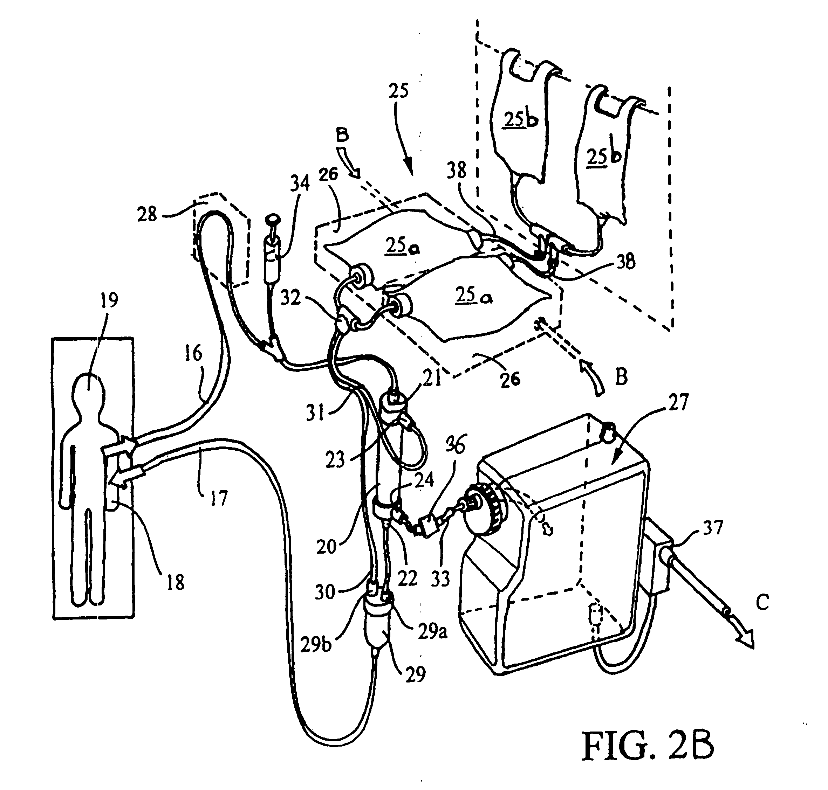

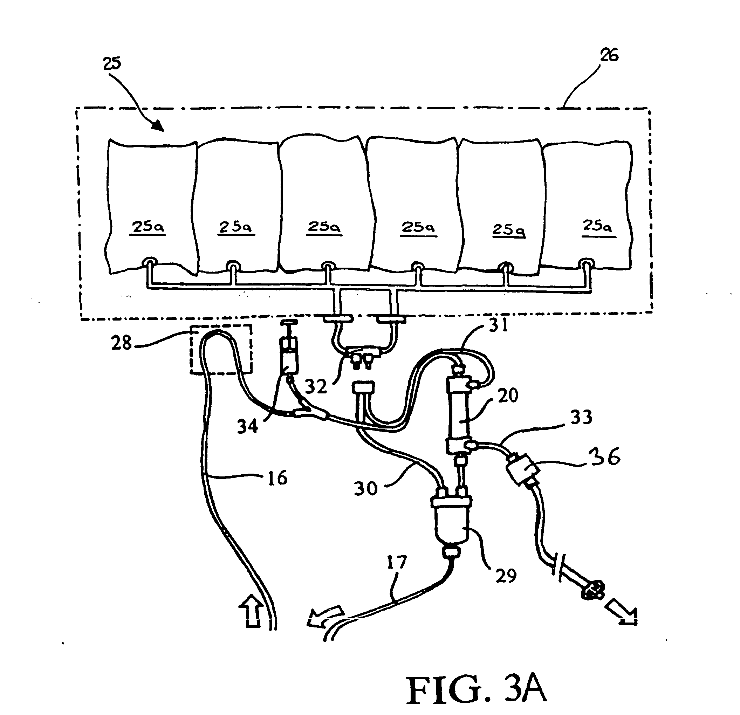

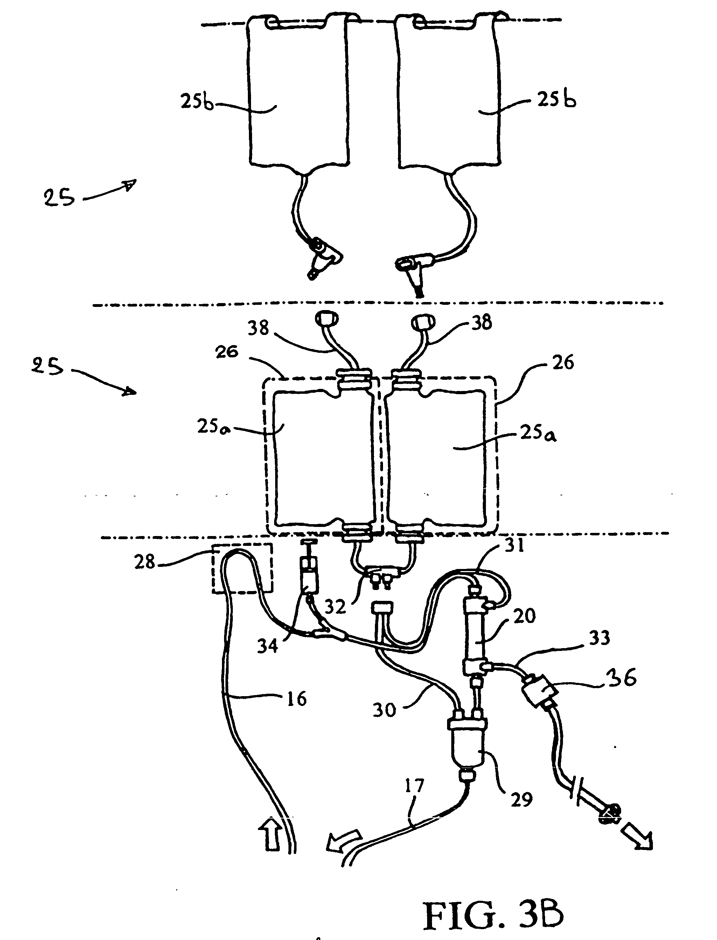

Nous allons décrire en détail les deux formes de réalisation préférées de la machine de dialyse 10 de l'invention correspondant respectivement à la dialyse chronique, en référence plus particulièrement aux figures 2A et 3A, et à la dialyse aiguë, en référence plus particulièrement aux figures 2B et 3B. Les composants identiques de ces deux formes de réalisation portent les mêmes références numériques dans les figures. La machine de dialyse 10 comporte principalement les composants suivants:

- un dialyseur 20, du type à fibres creuses ayant une première entrée 21 et une première sortie 22, une seconde entrée 23 et une seconde sortie 24;
- des moyens d'approvisionnement 25 en dialysat qui seront décrits plus en détail par la suite;
- au moins une enceinte 26 pouvant être mise sous une faible pression au moyen d'un gaz tel que de l'azote ou de l'air comprimé symboliquement représenté par la flèche B, cette enceinte contenant au moins partiellement lesdits moyens d'approvisionnement 25 en dialysat;
- un réservoir de récupération 27 du dialysat rejeté;
- la ligne artérielle 16 qui relie la fistule artérioveineuse implantée dans l'avant bras 18 du patient 19 à la première entrée 21 du dialyseur 20;
- une pompe péristaltique 28 montée sur la ligne artérielle 16 pour générer une circulation du sang du patient à travers le dialyseur 20 agissant comme un filtre;

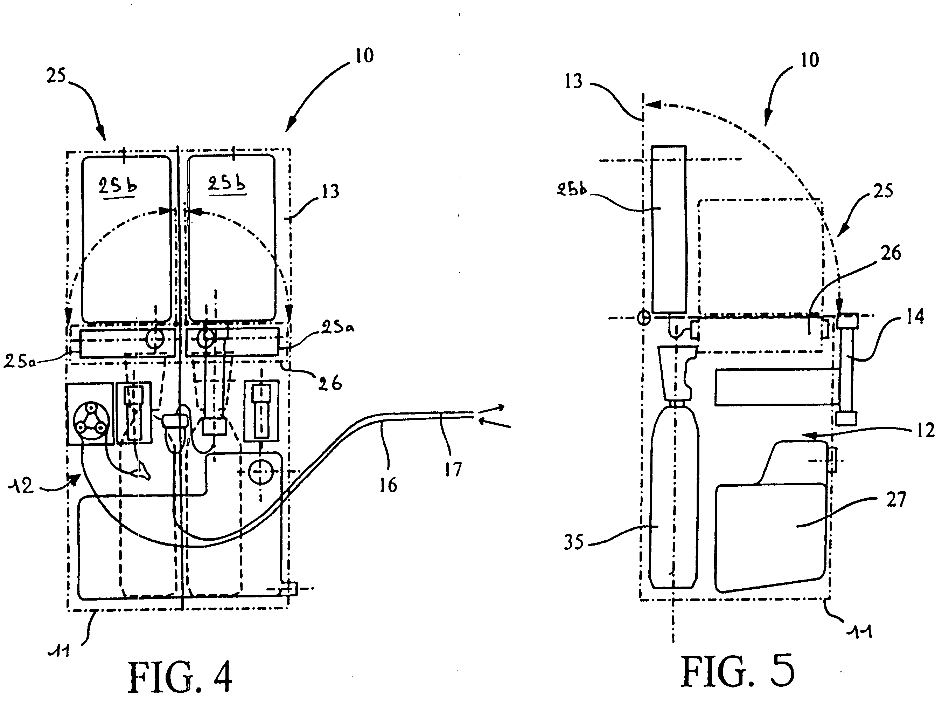

- la ligne veineuse 17 connectée à ladite fistule artérioveineuse implantée dans l'avant bras 18 du patient 19 et à la sortie d'un piège à bulles 29, dont une première entrée 29a est raccordée à la première sortie 22 du dialyseur 20;
- une ligne d'amenée du dialysat 31 connectée entre un collecteur ou séparateur 32 raccordé auxdits moyens d'approvisionnement 25 en dialysat et la seconde entrée 23 du dialyseur 20;
- une ligne d'évacuation 33 qui connecte la seconde sortie 24 du dialyseur 20 au récipient de récupération 27;
- un pousse seringue 34 ou un dispositif commandé automatiquement ayant des fonctions équivalentes pouvant contenir un médicament, tel que par exemple un anticoagulant, et branché en dérivation sur la ligne artérielle 16;
- un réservoir de gaz 35 tel qu'une bouteille d'azote (voir figure 5) ou un compresseur d'air pour assurer la mise sous pression de l'intérieur de l'enceinte 26 et par conséquent des moyens d'approvisionnement 25 en dialysat stérile;
- un dispositif volumétrique 36, de préférence une pompe de dialysat, connecté sur la ligne d'évacuation 33;
- un dispositif de rejet 37 du dialysat à l'égout, schématiquement représenté par la flèche C.

La ligne d'amenée 31 du dialysat au dialyseur 20 comporte une importante restriction de débit qui génère une perte de charge. C'est cette perte de charge qui permet de définir le débit du dialysat. Cette restriction est de préférence constituée d'un tube armé calibré, de section constante, constituant un étranglement sur une longueur prédéterminée, relativement importante.

Le fait que cette restriction soit présente sur une grande longueur du tube permet de stabiliser le débit et d'éviter des disparités dues à des imperfections résultant notamment de l'extrusion du tube.

La machine de dialyse 10 destinée à une dialyse chronique, figures 2A et 3A, comporte, dans l'exemple représenté, une seule enceinte 26. On connaît très précisément la durée de l'intervention et on connaît le volume de dialysat requis. Etant donné que ce volume est relativement réduit, de l'ordre de 25 à 30 litres, l'enceinte 26 peut être configurée de telle manière qu'elle puisse contenir tout le volume nécessaire à la dialyse. Les moyens d'approvisionnement 25 en dialysat stérile comportent, dans l'exemple représenté, six poches souples 25a de relative faible capacité, par exemple de 5 litres chacune, disposées à l'intérieur de l'enceinte 26 et connectées au collecteur 32 raccordé à la ligne d'amenée du dialysat 31. La pression dans l'enceinte 26 est directement appliquée aux poches 25a et l'enceinte est maintenue à une température appropriée pour que le dialysat stérile soit sensiblement à 37°C.

Dans le cas de la dialyse aiguë, figures 2B et 3B, le traitement s'effectue en continu et peut durer des semaines. Le dialysat doit être à disposition de manière permanente. A cet effet, la machine de dialyse 10 comporte, dans l'exemple représenté, deux enceintes 26, pouvant être mises sous pression séparément par les moyens de mise sous pression 35, symboliquement représenté par les deux flèches B. Les moyens d'approvisionnement 25 en dialysat stérile comportent d'une part deux poches souples 25a, de relative faible capacité (par exemple 5 litres chacune), disposées chacune respectivement à l'intérieur d'une enceinte 26 et qui sont couplées par l'intermédiaire du collecteur 32, et d'autre part deux poches 25b, de relative faible capacité (par exemple 5 litres chacune), disposées à l'extérieur des enceintes 26, chacune d'elles étant connectée respectivement à une poche 25a par un conduit de raccordement 38. Les poches 25b constituent une réserve et sont portées par un support solidaire du bâti 11. Ce support est constitué par le couvercle 13 de la machine 10.

Cet agencement particulier facilite les manipulations et évite l'ouverture des enceintes 26, qui peuvent être sous pression. En effet, les poches 25a sont maintenues à demeure dans leur enceinte et le réapprovisionnement se fait au moyen des poches 25b qui sont à l'extérieur des enceintes et sont remplacées régulièrement, par une infirmière par exemple. La présence des deux poches 25a dans les enceintes permet le remplissage alterné par simple gravité à partir des poches de réserve 25b, moyennant toutefois la présence de systèmes de pinçage alternatif des conduits, couramment appelés des « clamps » et la dépressurisation alternée de l'une des enceintes, pour charger successivement et alternativement les poches 25a à partir des poches 25b contenant la réserve. Ceci permet d'alimenter en continu la ligne d'amenée du dialysat 31 à partir de l'une des poches 25a disposée dans l'enceinte 26 sous pression.

La machine de dialyse 10 telle que décrite précédemment pour la dialyse chronique et pour la dialyse aiguë peut être configurée pour une utilisation destinée à pratiquer une technique appelée hémodiafiltration, dans laquelle le dialysat stérile sert à la fois de liquide d'échange avec le sang, au niveau du dialyseur 20, et de liquide de substitution, directement introduit dans le circuit sanguin du patient. A cet effet, le collecteur 32, couplé aux poches 25a de dialysat stérile, est raccordé par l'intermédiaire d'une ligne d'amenée 30 en liquide de substitution, c'est-à-dire du dialysat stérile, à une deuxième entrée 29b du piège à bulles 29, dont la sortie est connectée à la ligne veineuse 17 raccordée à la fistule artérioveineuse implantée dans l'avant bras 18 du patient 19. Il est à noter que cette ligne d'amenée 30 peut être maintenue à demeure sur la machine de dialyse 10. Celle-ci est obturée par un dispositif de pinçage (non représenté sur les figures), couramment appelé un "clamp", quand la machine n'est pas utilisée pour pratiquer une hémodiafiltration.

Afin de limiter les équipements de contrôle électronique tout en conservant un caractère fiable, simple et sûr de la machine de dialyse 10, les tubulures principales, à savoir les lignes 30 et 31 qui relient respectivement les moyens d'approvisionnement 25 en dialysat stérile au piège à bulle 29 et au dialyseur 20 sont calibrées de manière précise pour que les débits autorisés soient parfaitement connus pour une pression déterminée régnant à l'intérieur de l'enceinte 26.

Dans le cas d'une hémodiafiltration, pendant la durée de la dialyse, soit environ deux heures et demi, la ligne 31 délivre environ 25 litres de dialysat et la ligne 30 délivre environ 5 litres de liquide de substitution dans la ligne veineuse 17 du patient 19. Cette méthode permet d'améliorer la performance d'épuration du sang grâce à une grande convection.

La machine de dialyse 10 retire à travers la membrane du dialyseur 20 une certaine quantité d'eau, soit un à deux litres par jour, que le patient ne peut pas éliminer. Ceci est effectué grâce à la pompe de dialysat 36, qui est une pompe dont le débit peut être ajusté, et qui en combinaison avec les moyens de mise sous pression 35 de l'enceinte 26, permet de générer une pression transmembranaire (TMP) déterminée, c'est-à-dire la différence entre la pression côté circuit sanguin et la pression côté circuit dialysat dans le dialyseur 20. Cette pression transmembranaire est ajustée de manière à éliminer la quantité d'eau requise.

La pression transmembranaire dépend également de la perte de charge de la membrane de dialyse utilisée et de la perte de charge dans la ligne d'amenée 31 du dialysat, qui est connectée entre les poches 25a contenues dans l'enceinte 26 et le dialyseur 20. Il est indispensable que la ligne 31 ait une perte de charge telle que l'eau passe plus facilement à travers la membrane sous l'effet de la pression transmembranaire que le dialysat ne passe des poches 25a vers le dialyseur 20. En d'autres termes, on crée une perte de charge dans la ligne 31 suffisamment importante pour que, quelle que soit la pression transmembranaire générée par la pompe de dialysat 36 et la pression intérieure de l'enceinte 26 sur le dialysat, le surplus de dialysat demandé ne puisse venir que de la membrane et non de l'enceinte 26. La pression dans l'enceinte 26 doit être suffisante pour que le débit du dialysat soit de l'ordre de 100 à 500 ml/minute dans la ligne 31, de préférence entre 100 et 250 ml/minute et avantageusement de l'ordre de 150 ml/minute. Dans le cas de l'hémodiafiltration, les 5 litres de dialysat stérile, injectés dans la ligne veineuse 17 sont retirés du coté dialysat en créant une pression transmembranaire appropriée, par exemple en créant une dépression au moyen de la pompe de dialysat 36. Cette pompe de dialysat est un dispositif volumétrique qui est totalement fiable pour assumer sa fonction.

La plupart des composants de la machine sont disposés dans le logement 12 du bâti 11, comme le montrent les figures 4 et 5. Pour une dialyse aiguë, le couvercle 13 de la machine 10 est relevé en cours d'utilisation et les poches de réserve 25b sont suspendues verticalement. Un dispositif électronique de commande est également disposé à l'intérieur de ce logement 12. Il est couplé au tableau de commande 14, illustré également par la figure 1.

La machine de dialyse 10 pourrait comporter des moyens de pesée agencés pour déterminer l'ultrafiltration du patient selon l'une ou l'autre des deux méthodes suivantes connues en soi, dans lesquelles:

- $P_1$ est le poids de dialysat mesuré par les moyens de pesée à l'entrée du dialyseur,

- P2 est le poids de dialysat mesuré à la sortie du dialyseur, et

- UF est l'ultrafiltration du patient
  a)

- on détermine l'ultrafiltration du patient par l'équation UF= $\Delta P$ = P2 - P1
  b)

- on mesure $P_1 + P_2$ à l'instant $t_1$, par exemple au début de la dialyse,

- on mesure $P_1 + P_2$ à l'instant $t_2$, par exemple à la fin de la dialyse,

- on calcule ensuite l'ultrafiltration du patient qui est égale à la différence des sommes $P_1 + P_2$ au début et à la fin de la dialyse

$$UF= (Pi + P_2)_{t2} - (P_1 + P_2)_{t1}$$

Ces pesées permettent de contrôler simplement et en toute sécurité l'efficacité de la dialyse en cours ou la fin de la séance de traitement.

La forme et les dimensions de la machine ne sont pas limitées à celles déductibles des figures. Une électronique de commande simple et fiable constitue un élément indispensable pour permettre à un patient de pratiquer l'auto-dialyse journalière sans risques et sans difficultés.

Les avantages de cette machine sont dus à sa simplicité de construction et à sa grande fabilité, ce qui la rend peu coûteuse et sûre à l'utilisation. En outre, elle présente une grande souplesse d'utilisation et se prête à tous les modes de dialyse: hémodialyse, hémodiafiltration, hémofiltration pré et/ou postdilution.

**Revendications**

1. Machine de dialyse comportant un dialyseur (20) ayant une première entre (21) et une première sortie (22) du circuit sanguin, des moyens d'approvisionnement (25) en dialysat, une ligne artérielle (16) susceptible d'etre connectée entre une fistule artérioveineuse du patient et ladite première entrée du dialyseur, une ligne veineuse (17) connectée entre ladite fistule artérioveineuse et ladite première sortie du dialyseur, une pompe (28) couplée à ladite ligne artérielle, une ligne (31) d'amenée du dialysat, connectée entre lesdits moyens d'approvisionnement en dialysat et une seconde entrée (23) du dialyseur, et une ligne d'évacuation (33) du dialysat connectée entre un récipient de récupération (27) du dialysat et une seconde sortie (24) du dialyseur, **caractérisée en ce que** lesdits moyens d'approvisionnement en dialysat (25) comportent au moins une enceinte (26) dans laquelle est disposée au moins une poche (25a) contenant du dialysat stérile, cette poche étant connectée à ladite ligne d'amenée du dialysat (31) vers le dialyseur (20), et des moyens de mise sous pression de ladite poche (25a) pour provoquer l'écoulement du dialysat dans cette ligne d'amenée (31), **en ce que** ladite ligne d'amenée de dialysat (31) comporte une restriction de débit calibrée agencée pour générer une perte de charge déterminée de telle façon que, pour une pression déterminée dans l'enceinte (26), le débit et la pression du dialysat à ladite seconde entrée (23) du dialyseur (20) ont une valeur donnée constante, et **en ce que** la machine comporte un dispositif volumétrique (36) connecté sur la ligne d'évacuation (33) à la seconde sortie (24) du dialyseur (20), ce dispositif volumétrique (36) étant agencé pour générer, en combinaison avec lesdits moyens de mises sous pression (35) de l'enceinte (26), une pression transmembranaire (TMP) déterminée dans le dialyseur.

2. Machine de dialyse selon la revendication 1, **caractérisée en ce que**, pour une dialyse aiguë, lesdits moyens d'approvisionnement (25) comportent au moins deux enceintes (26) contenant chacune au moins une poche (25a) de dialysat stérile, les deux poches (25a) pouvant être mises sous pression séparément par lesdits moyens de mise sous pression, et au moins deux poches de réserve (25b) de dialysat stérile disposées à l'extérieur desdites enceintes (26) et connectées chacune à l'une desdites poches (25a) respectivement par un conduit (38).

3. Machine de dialyse selon la revendication 2, **caractérisée en ce qu'**elle comporte un système de pinçage alternatif des conduits (38).

4. Machine de dialyse selon la revendication 1 ou 2, **caractérisée en ce que**, pour une utilisation destinée à pratiquer de l'hémodiafiltration, lesdits moyens d'approvisionnement (25) comprennent au moins une poche (25a) de dialysat stérile logée dans une enceinte (26) et connectée par l'intermédiaire d'une ligne d'amenée (30) à ladite ligne veineuse (17).

5. Machine de dialyse selon la revendication 1, **caractérisée en ce que** lesdits moyens de mise sous pression comportent au moins un réservoir de gaz (35) raccordé à ladite enceinte (26).

6. Machine de dialyse selon la revendication 1, **caractérisée en ce que** lesdits moyens de mise sous pression comportent au moins un compresseur d'air.

7. Machine de dialyse selon la revendication 1, **caractérisée en ce que** la ligne d'amenée du dialysat (31) au dialyseur (20) est calibrée pour que, pour une pression déterminée dans l'enceinte (26), le débit de dialysat ait une valeur sensiblement comprise entre 100 et 500 ml/minute et de préférence comprise entre 100 et 250 ml/minute.

8. Machine de dialyse selon la revendication 1, **caractérisée en ce que** la ligne d'amenée du dialysat (31) est constituée au moins en partie d'un tube armé calibré de section constante sur une longueur prédéterminée.

9. Machine de dialyse selon la revendication 4, **caractérisée en ce que** les moyens de mise sous pression maintiennent une pression intérieure de l'enceinte (26) suffisante pour provoquer l'écoulement du dialysat dans les lignes d'amenée (30 et 31) respectivement à un piège à bulles (29) et au dialyseur (20).

10. Machine de dialyse selon la revendication 9, **caractérisée en ce que** les lignes d'amenée (30 et 31) sont calibrées pour que, pour une pression déterminée dans l'enceinte (26), les débits aient une valeur donnée constante.

11. Machine de dialyse selon la revendication 1, **caractérisée en ce que** ledit dispositif volumétrique (36) est une pompe de dialysat dont le débit peut être ajusté.

12. Machine de dialyse selon la revendication 1, **caractérisée en ce qu'**elle comporte un bâti (11) portant un logement (12) contenant ledit dialyseur (20), au moins une enceinte (26) contenant au moins une poche (25a) de dialysat stérile, ladite ligne artérielle (16) agencée pour être connectée entre une artère du patient (19) et ladite première entrée (21) du dialyseur (20), ladite ligne veineuse (17) agencée pour être connectée entre une veine du patient (19) et ladite première sortie (22) du dialyseur (20), ladite pompe (28) couplée à ladite ligne artérielle (16), ladite ligne d'amenée (31) du dialysat stérile, connectée entre ladite poche de dialysat stérile (25a) et la seconde entrée (23) du dialyseur (20), ledit récipient de récupération (27) du dialysat, ladite ligne d'évacuation (33) du dialysat, connectée entre ladite seconde sortie (24) du dialyseur (20) et ledit récipient de récupération (27), et lesdits moyens de mise sous pression de ladite poche (25a) de dialysat stérile logée dans ladite enceinte (26), et un couvercle rabattable (13) attaché à ce bâti (11).

13. Machine de dialyse selon les revendications 2 et 12, **caractérisée en ce que** ledit logement (12) renferme les deux enceintes (26) contenant chacune au moins une poche (25a) de dialysat stérile, et **en ce que** la machine de dialyse comporte un support solidaire du bâti (11) et qui porte les poches de réserve (25b) de dialysat stérile.

14. Machine de dialyse selon la revendication 13, **caractérisée en ce que** ledit support des poches de réserve (25b) est constitué par ledit couvercle (13).

## Patentansprüche

1. Dialysemaschine umfassend einen Dialysator (20), der einen ersten Eingang (21) und einen ersten Ausgang (22) des Blutkreislaufs aufweist, Mittel (25) zur Versorgung mit Dialysat, eine arterielle Leitung (16), die geeignet ist, zwischen einer arteriovenösen Fistel des Patienten und dem ersten Eingang des Dialysators angeschlossen zu werden, eine venöse Leitung (17), die zwischen der genannten arteriovenösen Fistel und dem ersten Ausgang des Dialysators angeschlossen ist, eine Pumpe (28), die an die arterielle Leitung gekoppelt ist, eine Leitung (31) zum Zuführen des Dialysats, die zwischen den Mitteln zur Versorgung mit Dialysat und einem zweiten Eingang (23) des Dialysators angeschlossen ist, sowie eine Leitung (33) zum Abführen des Dialysats, die zwischen einem Behältnis (27) zur Rückgewinnung des Dialysats und einem zweiten Ausgang (24) des Dialysators angeschlossen ist, **dadurch gekennzeichnet, dass** die Mittel (25) zur Versorgung mit Dialysat wenigstens einen Behälter (26), in dem wenigstens eine steriles Dialysat enthaltende Tasche (25a) angeordnet ist, wobei diese Tasche an die Leitung (31) zum Zuführen des Dialysats zu dem Dialysator (20) angeschlossen ist, sowie Mittel zum Unterdrucksetzen der genannten Tasche (25a) aufweisen, um das Einströmen des Dialysats in diese Zuführleitung (31) zu bewirken, dass die genannte Leitung (31) zum Zuführen des Dialysats eine kalibrierte Durchflussmengendrossel aufweist, die angeordnet ist, um einen bestimmten Druckverlust zu erzeugen, derart, dass bei einem bestimmten Druck in dem Behälter (26) die Durchflussmenge und der Druck des Dialysats am zweiten Eingang (23) des Dialysators (20) einen gegebenen konstanten Wert haben, und dass die Maschine eine volumetrische Vorrichtung (36) aufweist, die an dem zweiten Ausgang (24) des Dialysators (20) an die Abführleitung (33) angeschlossen ist, wobei diese volumetrische Vorrichtung (36) angeordnet ist, um in Kombination mit den genannten Mitteln (35) zum Unterdrucksetzen des Behälters (26) in dem Dialysator einen bestimmten transmembranen Druck (TMP) zu erzeugen.

2. Dialysemaschine nach Anspruch 1, **dadurch gekennzeichnet, dass** für eine akute Dialyse die Versorgungsmittel (25) wenigstens zwei Behälter (26) aufweisen, die jeweils wenigstens eine Tasche (25a) mit sterilem Dialysat enthalten, wobei die zwei Taschen (25a) durch die Mittel zum Unterdrucksetzen getrennt unter Druck gesetzt werden können, sowie wenigstens zwei Reservetaschen (25b) mit sterilem Dialysat, die außerhalb der Behälter (26) angeordnet und jeweils über eine Leitung (38) mit jeweils einer der genannten Taschen (25a) verbunden sind.

3. Dialysemaschine nach Anspruch 2, **dadurch gekennzeichnet, dass** sie ein System zum abwechselnden Abklemmen der Leitungen (38) umfasst.

4. Dialysemaschine nach Anspruch 1 oder 2, **dadurch gekennzeichnet, dass** für eine Verwendung zur Durchführung einer Hämodiafiltration die genannten Versorgungsmittel (25) wenigstens eine Tasche (25a) mit sterilem Dialysat aufweisen, die in einem Behälter (26) angeordnet und mittels einer Zuführleitung (30) an die genannte venöse Leitung (17) angeschlossen ist.

5. Dialysemaschine nach Anspruch 1, **dadurch gekennzeichnet, dass** die genannten Mittel zum Unterdrucksetzen wenigstens einen Gasbehälter (35) umfassen, der mit dem Behälter (26) verbunden ist.

6. Dialysemaschine nach Anspruch 1, **dadurch gekennzeichnet, dass** die genannten Mittel zum Unterdrucksetzen wenigstens einen Luftverdichter aufweisen.

7. Dialysemaschine nach Anspruch 1, **dadurch gekennzeichnet, dass** die Leitung (31) zum Zuführen des Dialysats zum Dialysator (20) kalibriert ist, damit bei einem bestimmten Druck in dem Behälter (26) die Dialysatdurchflussmenge einen Wert hat, der im Wesentlichen zwischen 100 und 500 ml/Minute und vorzugsweise zwischen 100 und 250 ml/Minute liegt.

8. Dialysemaschine nach Anspruch 1, **dadurch ge-** **kennzeichnet, dass** die Dialysatzuführleitung (31) wenigstens teilweise von einer kalibrierten verstärkten Röhre mit konstantem Querschnitt über eine vorbestimmte Länge gebildet ist.

9. Dialysemaschine nach Anspruch 4, **dadurch gekennzeichnet, dass** die Mittel zum Unterdrucksetzen einen Innendruck des Behälters (26) aufrechterhalten, der ausreicht, um zu bewirken, dass das Dialysat in den Zuführleitungen (30 und 31) zu einer Blasenfalle (29) bzw. zu dem Dialysator (20) strömt.

10. Dialysemaschine nach Anspruch 9, **dadurch gekennzeichnet, dass** die Zuführleitungen (30 und 31) kalibriert sind, damit die Durchflussmengen bei einem bestimmten Druck in dem Behälter (26) einen gegebenen konstanten Wert haben.

11. Dialysemaschine nach Anspruch 1, **dadurch gekennzeichnet, dass** die volumetrische Vorrichtung (36) eine Dialysatpumpe ist, deren Fördermenge eingestellt werden kann.

12. Dialysemaschine nach Anspruch 1, **dadurch gekennzeichnet, dass** sie ein Gestell (11) aufweist, welches eine Aufnahme (12) trägt, enthaltend den Dialysator (20), wenigstens einen Behälter (26), der wenigstens eine Tasche (25a) mit sterilem Dialysat enthält, die arterielle Leitung (16), welche angeordnet ist, um zwischen einer Arterie des Patienten (19) und dem ersten Eingang (21) des Dialysators (20) angeschlossen zu werden, die venöse Leitung (17), die angeordnet ist, um zwischen einer Vene des Patienten (19) und dem ersten Ausgang (22) des Dialysators (20) angeschlossen zu werden, die an die arterielle Leitung (16) gekoppelte Pumpe (28), die Leitung (31) zum Zuführen des sterilen Dialysats, die zwischen der Tasche (25a) mit sterilem Dialysat und dem zweiten Eingang (23) des Dialysators (20) angeschlossen ist, das Behältnis (27) zur Rückgewinnung des Dialysats, die Leitung (33) zum Abführen des Dialysats, welche zwischen dem zweiten Ausgang (24) des Dialysators (20) und dem Rückgewinnungsbehältnis (27) angeschlossen ist sowie die Mittel zum Unterdrucksetzen der in dem Behälter (26) aufgenommenen Tasche (25a) mit sterilem Dialysat, und dass sie einen herunterklappbaren Deckel (13) aufweist, der an diesem Gestell (11) befestigt ist.

13. Dialysemaschine nach den Ansprüchen 2 und 12, **dadurch gekennzeichnet, dass** die genannte Aufnahme (12) beide Behälter (26) enthält, die jeweils wenigstens eine Tasche (25a) mit sterilem Dialysat enthalten, und dass die Dialysemaschine einen mit dem Gestell (11) fest verbundenen Halter umfasst, der die Reservetaschen (25b) mit sterilem Dialysat trägt.

**14.** Dialysemaschine nach Anspruch 13, **dadurch gekennzeichnet, dass** der Halter für die Reservetaschen (25b) von dem genannten Deckel (13) gebildet ist.

## Claims

**1.** A dialysis machine comprising a dialyzer (20) with a first blood circulation inlet (21) and a first blood circulation outlet (22), a dialysate supply means (25), an arterial line (16) able to be connected between an arteriovenous fistula in the patient and said first dialyzer inlet, a venous line (17) connected between said arteriovenous fistula and said first dialyzer outlet, a pump (28) coupled with said arterial line, a dialysate inlet line (31) connected between said dialysate supply means and a second dialyzer inlet (23), and a dialysate evacuation line (38) connected between a dialysate collection container (27) and a second dialyzer outlet (24), **characterized in that** said dialysate supply means (25) comprises at least one chamber (26) in which at least one pouch (25a) containing sterile dialysate is disposed, this pouch being connected to said dialysate inlet line (31) leading to the dialyzer (20), and a means for pressurizing said pouch (25a) to provoke the flow of dialysate into said inlet line (31), **in that** said dialysate inlet line (31) includes a flow restriction designed to generate a predetermined decrease in charge calculated so that for a given pressure in the chamber (26), the flow rate and the pressure of the dialysate at said second inlet (23) on the dialyzer (20) have a constant given value, and **in that** the machine comprises a volumetric device (36) connected to the evacuation line (33) at the second outlet (24) of the dialyzer (20), said volumetric device (36) being designed to generate, in combination with said means (35) for pressurizing the chamber (26), a predetermined transmembrane pressure (TMP) in the dialyzer.

**2.** A dialysis machine according to claim 1, **characterized in that**, during acute dialysis, said supply means (25) comprises at least two chambers (26) each containing at least one pouch (25a) of sterile dialysate, both said pouches (25a) capable of separate pressurization by said pressurization means and at least two reserve pouches (25b) of sterile dialysate located outside said chambers (26) and each respectively connected to one of said pouches (25a) with a conduit (38).

**3.** A dialysis machine according to claim 2, **characterized in that** it comprises a system for alternately clamping the conduits (38).

**4.** A dialysis machine according to claim 1 or 2, **char-**acterized in that**, when used to perform hemodiafiltration, said supply means (25) comprises at least one pouch (25a) of sterile dialysate located in a chamber (26) and connected by means of an inlet line (30) leading to said venous line (17).

**5.** A dialysis machine according to claim 1, **characterized in that** said pressurization means comprises at least one reservoir of gas (35) connected to said chamber (26).

**6.** A dialysis machine according to claim 1, **characterized in that** said pressurization means comprises at least one air compressor.

**7.** A dialysis machine according to claim 1, **characterized in that** the dialysate inlet line (31) leading to the dialyzer (20) is calibrated so that for a predetermined pressure in the chamber (26), the dialysate will flow at a rate ranging essentially from 100 to 500 ml/minute and preferably from 100 to 250 ml/minute.

**8.** A dialysis machine according to claim 1, **characterized in that** the dialysate inlet line (31) consists at least partially of a reinforced calibrated tube of constant section for a predetermined length.

**9.** A dialysis machine according to claim 4, **characterized in that** the pressurization means maintains an interior pressure within the chamber (26) that is sufficient to provoke the flow of dialysate through the inlet lines (30 and 31), respectively, to a bubble trap (29) and to the dialyzer (20).

**10.** A dialysis machine according to claim 9, **characterized in that** the inlet lines (30 and 31) are calibrated so that, for a predetermined pressure in the chamber (26), the flow rates will have a constant given value.

**11.** A dialysis machine according to claim 1, **characterized in that** said volumetric device (36) is a dialysate pump with an adjustable rate of flow.

**12.** A dialysis machine according to claim 1, **characterized in that** it comprises a frame (11) with a housing (12) containing said dialyzer (20), at least one chamber (26) containing at least one pouch (25a) of sterile dialysate, said arterial line (16) disposed to be connected between an artery of the patient (19) and said first inlet (21) of the dialyzer (20), said venous line (17) disposed to be connected between a vein of the patient (19) and said first outlet (22) of the dialyzer (20), said pump (28) coupled to said arterial line (16), said sterile dialysate inlet line (31) connected between said sterile dialysate pouch (25a) and the second inlet (23) on the dialyzer (20), said dialysate collection container (27), said dialysate evacuation line (33) connected between said second outlet (24) on the dialyzer (20) and said collection container (27), and said means for pressurizing said pouch (25a) of sterile dialysate housed in said chamber (26), and a folding cover (13) attached to this frame (11).

13. A dialysis machine according to claims 2 and 12, **characterized in that** said housing (12) encloses the two chambers (26), each containing at least one pouch (25a) of sterile dialysate, and **in that** the dialysis machine comprises a support integral with the frame (11) and which holds the reserve pouches (25b) of sterile dialysate.

14. A dialysis machine according to claim 13, **characterized in that** said support for the reserve pouches (25b) consists of said cover (13).

FIG. 1

FIG. 2A

FIG. 2B

FIG. 3A

FIG. 3B

FIG. 5

FIG. 4